# EUROPEAN PATENT APPLICATION

(11) **EP 3 695 845 A1**
(43) Date of publication of application: **19.08.2020**
(21) Application number: 20155846.7
(22) Date of filing: 11.05.2017
(51) Int. Cl.: A61K 35/14, A61K 35/16, A61K 31/728, A61P 1/16, A61P 3/10

(54) **ANTI-AGEING PHARMACEUTICAL PREPARATION**

(30) Priority: 17.08.2016 EP 16001807; 14.11.2016 WO PCT/EP2016/001887; 14.11.2016 WO PCT/EP2016/001888
(62) Divisional of application: 17728051.8
(71) Applicant: Orthogen AG, 40212 Düsseldorf (DE)
(72) Inventor: WEHLING, Peter, 40597 Düsseldorf (DE); REINECKE, Julio, 50733 Köln (DE)
(74) Representative: Hertzsch, Claudia

(57) **Abstract**

The present invention provides a pharmaceutical preparation for use by injection into the skin, wherein the pharmaceutical preparation is preparable by a production method comprising the steps of providing a liquid collected from an organism, which liquid comprises cellular constituents of blood, providing a vessel or containment means, said vessel or containment means having an internal surface, and contacting said liquid with said vessel or containment means, wherein
(a) said production method further comprises the step of incubating said liquid in said vessel or containment means for an incubation time, and optionally removing cellular constituents of said liquid after said incubation,
(b) said liquid comprises exosomes, and said production method further comprises the steps of concentrating said exosomes and optionally removing cellular constituents of said liquid after said concentration, or the step of isolating said exosomes, or
(c) said production method further comprises the step of avoiding incubation of said liquid, and the step of removing cellular constituents of said liquid contacted with said vessel or containment means.

## Description

The present invention relates to the field of ageing, in particular to a pharmaceutical preparation for use in the treatment of ageing and/or age-related disorders or for use as an anti-ageing agent.

Managing ageing of the human population and ageing-associated disorders is a global challenge. Many industrialised countries have an increasingly ageing population, and also in developing countries the proportion of elderly people is expected to rise steeply. On a biological level ageing may be defined as a progressive deterioration over time of an organism and its individual cells, tissues and organs. Such deterioration may lead to certain age-related disorders. It has been estimated that by 2050 the number of people aged 60 and older worldwide will approximately double as compared to its current figure of around 11%.

Several theories of ageing and associated frailty have been proposed. One theory focuses on a progressive accumulation of damage. Longevity and health at an advanced age appear to be influenced, amongst other factors, by a balance between damage caused to biomolecules (in particular DNA) and maintenance and repair systems. Therefore ageing has been proposed to be correlated to DNA damage, and certain age-associated disorders might be due to an excess of DNA damage or an impaired DNA repair. Therefore corresponding animal models of ageing have been developed, and widespread models include mice that have defects in Nucleotide Excision Repair (NER) and show a premature ageing phenotype as compared to wild-type mice. It is believed that the alterations in such mice and the accompanying disorders reflect normal ageing in mice, but also in other organisms such as humans. Oxidative damage to cells and cell components has also been implicated in ageing and certain disorders, and thus the lack of sufficient repair, such as the impaired expression or activity of antioxidant enzymes. Cellular senescence has also been linked to the shortening of telomeres, which may act as a molecular clock and thus might be the cause of certain disorders linked with senescence. Where cells become incapable of maintaining a sufficient rate of cell division, a lack of renewal of tissues might promote age-related disorders. Excessive cell death such as apoptosis may also contribute to ageing, senescence and associated disorders. Another ageing theory focuses on excessive (even if subtle) inflammation, which exerts harmful effects in advanced age. The immune system is also subject to senescence, one characteristic being "immunosenescence" associated with a reduced sensitivity to vaccination.

Although formerly ageing has been regarded as an inevitable natural process, this view has recently shifted in the scientific community. According to a more recent notion, it is fair to describe ageing as a disease. It is also often emphasised that ageing is the most common risk factor for a number of diseases. Consequently the treatment (including prophylaxis) of ageing or one of its causes is therapeutic. This is not only because ageing itself may nowadays be classified as a disease, but also already because treating a risk factor for a disease is a therapeutic goal in itself.

Niccoli and Partridge (2012) point out that age is the main risk factor for major debilitating and life-threatening conditions, including cancer, cardiovascular disease and neurodegeneration (Current Biology 22, R741), and that whereas "[c]urrent therapies target individual diseases in isolation; therapies targeted to the ageing process itself aim to cover many diseases simultaneously". A 2010 article by Kelland (http://www.reuters.com/article/us-ageing-disease-idUSTRE64I6HV20100520) quotes an expert opinion that ageing is the biggest risk factor common to all age-related diseases and it is a failure not to investigate "the common mechanism for all of them", but only the specific diseases.

Kelland (2010) also reports that ageing experts say it is time for a fresh look at ageing, which recognises it as a condition that can be manipulated, treated and delayed. Indeed, for instance an article written by Bulterijs et al. (2015) is titled "It is time to classify biological aging as a disease" (Frontiers in Genetics, 6, 205). The authors argue that ageing is caused by the decrease in the force of selection against alleles with deleterious effects later in life, that ageing is thus the consequence of evolutionary neglect and not intent, and if it serves no purpose, the notion of ageing as a natural process might be mistaken. The authors conclude that ageing should be seen as a disease, albeit as a disease that is a universal and multisystemic process. Dr. Zhavoronkov, CEO of Insilico Medicine, believes that ageing should be considered a disease and says that describing ageing as a disease creates incentives to develop treatments. In this context it is interesting to note that in 2014 the "Palo Alto Longevity Prize" has been established, whose declared goal is to "hack the code of life and cure aging". According to one of the advisers on the prize board, Prof. Kim, ageing is simply a medical problem for which a solution can be found. One notable proponent of the notion that ageing is curable is the gerontologist and author Dr. de Grey, CSO of the SENS Research Foundation, who takes the view that scientists today are too focused on the diseases of ageing and not ageing itself. An article by Musa in the November 1, 2016 issue of The Scientist magazine contains the statement that ageing is "just another disease", no longer considered an inevitability and should be treated like a chronic condition.

Ultraviolet (UV) radiation is inter alia present in sunlight. It has a wavelength below that of visible light. Biologically, the UV-A band (defined by ISO-21348 as 315 to 400 nm), the most long-wave part of the UV spectrum, and the neighbouring UV-B band (280 to 315 nm) are most important, whereas UV radiation having a shorter wavelength (UV-C) is practically absorbed by the ozone layer and the earth's atmosphere. UV radiation is able to trigger chemical reactions. Whereas it has certain beneficial actions on the human body, it is also dangerous since it causes damage, in particular to the skin and the eyes. UV radiation (all bands) is known to damage collagen and elastin fibres, which gives rise to skin ageing (photoageing), whose signs may include loose skin, dryness and/or wrinkling.

In the DNA, UV-B radiation causes the formation of pyrimidine dimers (in particular thymine dimers), a process that is also known as direct DNA damage. The formed lesions alter the DNA structure and may be repaired by a mechanism known as Nucleotide Excision Repair. If unrepaired, the lesions are mutagenic. UV radiation also causes the production of reactive oxygen species/free radicals, which gives rise to oxidative damage, a process known as indirect DNA damage. Both direct and indirect DNA damage contribute to the formation of cancer. It is therefore apprehensible that broad-spectrum UV radiation is recognised as a carcinogen by the World Health Organisation.

One of the features of the immune system is an interplay between T cells and macrophages. In this regard, two major types of immune processes are commonly distinguished, Type 1 and Type 2. In the Type 1 processes, Type 1 T helper cells (Tₕ1 cells) and Type 1 macrophages (M1) are involved, and these processes play a major role in the cellular immune response and in the pathophysiology of inflammatory processes. The Type 2 processes involve Type 2 T helper cells (Tₕ2 cells) and Type 2 macrophages (M2) and play a role in anti-inflammatory and/or regenerative processes such as wound healing and tissue repair, besides their role in the humoral immune response. Typical cytokines associated with Type 1 processes are IFN-γ and IL-2. Type 1 immune response maximise the cellular killing ability. Where there is a (chronic) preponderance of Type 1 immune processes, damage to the organism may occur, e.g. when directed to autoantigens type 1 diabetes, and more generally ageing may be accelerated due to excessive and/or chronic inflammation. The preponderance of Type 1 immune processes may e.g. be a preponderance of Type 1 over Type 2 immune processes. Such an imbalance in immune processes might be countered by promoting other immune processes (e.g. Type 2) or inhibiting Type 1 immune processes, facilitating the resolution of inflammation.

In neurodegeneration, such as in Alzheimer's disease, Parkinson's disease and multiple sclerosis a role of macrophages (such as microglia) has been described.

Shifting the balance from Type 1 immune responses to Type 2 immune responses might be particularly interesting in nervous system or skin disorders.

Cellular senescence is a phenomenon that leads to the inability of isolated cells to divide perpetually and makes them arrest after a certain number of divisions. For the detection of cellular senescence a cellular staining assay that detects senescence-associated β-galactosidase activity is commonly used (see e.g. Dimri et al. (1995) PNAS 92, 9363 to 9367). Cells exhibiting a senescent phenotype may interfere with vital functions of a whole organism and thus lead to certain disorders. The senescence of a whole organism is accompanied by an increased risk of certain disorders (such as diseases, complications and conditions). It is found that the incidence of certain disorders increases with age in the greater than linear fashion, for example exponentially. As mentioned above, ageing is the main risk factor for a number of major diseases.

Common specific disorders correlated with age are atherosclerosis, cardiovascular disease, cancer, hearing deficits, vision deficits, cataracts, retinal degeneration, e.g. macular degeneration, osteoporosis, type 2 diabetes, hypertension, liver failure, cachexia, kyphosis, gait disorders, tremors, ataxia, dystonia, reduced grip strength, muscle wasting and hair greying. Age also promotes neurodegeneration and similar disorders, such as mild cognitive impairment, Alzheimer's disease, cerebrovascular disease, Parkinson's disease and amyotrophic lateral sclerosis.

It is a widespread desire to extend the lifespan and/or the healthspan, i.e. the life period during which one is generally healthy and free from serious disease. Whereas a few compounds or compositions have been suggested for this purpose or for the treatment of the above-mentioned disorders in the prior art, their efficacy and/or tolerability is not always clear. Thus there is a need for alternative treatments.

It is the problem of the present invention to provide a novel anti-ageing means or means for treating, preventing, inhibiting or mitigating ageing or one or more of the above-mentioned disorders, or for interfering with one or more of the biological causes of ageing or of such disorders. Preferably, such anti-ageing is directed to the skin, such ageing is skin ageing or such a disorder is a skin disorder, such as a disorder that can be assessed by a suction elasticity measurement (e.g. by using a Cutometer®). Advantageously, such a means is fast and easy to produce and is cost effective. Also advantageously such a means should have good body compatibility.

The above statements, and any description of exemplified embodiments herein, do not constitute any waiver of certain embodiments or features.

This problem is solved by a pharmaceutical preparation for use by injection into the skin, wherein the pharmaceutical preparation is preparable by a production method comprising the steps of providing a liquid collected from an organism, which liquid comprises cellular constituents of blood, providing a vessel or containment means, said vessel or containment means having an internal surface, and contacting said liquid with said vessel or containment means, wherein
(a) said production method further comprises the step of incubating said liquid in said vessel or containment means for an incubation time, and optionally removing cellular constituents of said liquid after said incubation,
(b) said liquid comprises exosomes, and said production method further comprises the steps of concentrating said exosomes and optionally removing cellular constituents of said liquid after said concentration, or the step of isolating said exosomes, or
(c) said production method further comprises the step of avoiding incubation of said liquid, and the step of removing cellular constituents of said liquid contacted with said vessel or containment means.

Said production method has three alternatives (a), (b), and (c) and is in the following referred to as the "adjusted production method of the present specification".

The present invention may also be described as a method of treating a patient in need of such treatment by injection of a pharmaceutical preparation into the skin, wherein the pharmaceutical preparation is preparable by a production method comprising the steps of providing a liquid collected from an organism, which liquid comprises cellular constituents of blood, providing a vessel or containment means, said vessel or containment means having an internal surface, and contacting said liquid with said vessel or containment means, wherein
(a) said production method further comprises the step of incubating said liquid in said vessel or containment means for an incubation time, and optionally removing cellular constituents of said liquid after said incubation,
(b) said liquid comprises exosomes, and said production method further comprises the steps of concentrating said exosomes and optionally removing cellular constituents of said liquid after said concentration, or the step of isolating said exosomes, or
(c) said production method further comprises the step of avoiding incubation of said liquid, and the step of removing cellular constituents of said liquid contacted with said vessel or containment means.

Additionally, the present invention may also be described as a method of preparing a pharmaceutical preparation for use by injection into the skin, wherein the method comprises the steps of providing a liquid collected from an organism, which liquid comprises cellular constituents of blood, providing a vessel or containment means, said vessel or containment means having an internal surface, and contacting said liquid with said vessel or containment means, wherein
(a) said production method further comprises the step of incubating said liquid in said vessel or containment means for an incubation time, and optionally removing cellular constituents of said liquid after said incubation,
(b) said liquid comprises exosomes, and said production method further comprises the steps of concentrating said exosomes and optionally removing cellular constituents of said liquid after said concentration, or the step of isolating said exosomes, or
(c) said production method further comprises the step of avoiding incubation of said liquid, and the step of removing cellular constituents of said liquid contacted with said vessel or containment means.

The following statements apply irrespective of whether the present invention is described as a pharmaceutical preparation for use by injection into the skin, as a method of treating a patient, or as a method of preparing a pharmaceutical preparation for use by injection into the skin.

In the following "the production method of the present specification" refers to a production method comprising the following steps: providing a blood sample collected from an organism and a vessel or container, contacting said blood sample with said vessel or a container, and incubating said blood sample in said vessel or container, wherein said blood sample is (1) a whole blood sample or (2) a whole blood sample from which erythrocytes have been depleted.

"Ageing" preferably refers to the accumulation of changes in an organism over time, physical changes and biological ageing being particularly preferred.

The term "anti-ageing" preferably refers to delaying, retarding, lessening, halting and/or reversing the effects of ageing (especially on the skin). This term may also refer to delaying, retarding, lessening, halting and/or reversing biological ageing (especially of the skin).

"Injection" includes any administration that uses one or more sharp objects (such as needles) to allow the pharmaceutical preparation to cross the skin surface. Preferably, the skin surface is penetrated by a hollow needle through which the pharmaceutical preparation is administered.

A "cellular constituent of blood" means any cellular constituent of whole blood, whether present in large or small amounts.

Preferably the "liquid" is a blood sample. In an even more preferred embodiment the liquid is a blood sample which is (1) a whole blood sample or (2) a whole blood sample from which cells have been depleted. In this context, the cells that have been depleted are preferably selected from erythrocytes, leukocytes (in particular neutrophils, eosinophils, basophils, lymphocytes, B cells, T cells, NK cells and/or monocytes) and platelets. More preferably the cells that have been depleted are erythrocytes.

"Containment means" refers to one or more containment means. The vessel or containment means may be the same as that/those or different than that/those in which said liquid has been collected. A preferred meaning of "a vessel or containment means" is "a container".

The "internal surface" of the vessel or containment means refers to the internal surface of the vessel or containment means itself. In particular, the surface of any macroscopic particles, microscopic particles or nanoparticles that might be contained in the vessel or containment means is not considered.

"Incubation" or "incubating" preferably refers to (i) an incubation with a duration of at least 2 min, 3 min, 4 min, 5 min, 7 min, 10 min, 15 min, 20 min, 25 min, 30 min, 35 min, 40 min, 45 min, 50 min, 55 min, 1 h, 1.5 h, 2 h, 2.5 h, 3 h, 3.5 h, 4 h, 4.5 h, 5 h, 5.5 h or 6 h and/or (ii) an incubation after which a measurable change in a certain parameter has occurred with respect to the value before incubation and/or the normal value of that parameter (such as the concentration of a cytokine, the concentration of a cytokine antagonist, in particular IL-1Ra, the concentration of exosomes and/or the concentration of a growth factor).

Where embodiments of the present invention are described as "containing" or "comprising" certain subject matter, e.g. methods steps, constituents or other features, it is understood that preferred embodiments consist of said subject matter, except where the context dictates otherwise.

It is understood that "treatment" also includes prophylaxis.

According to a preferred embodiment, the present invention therefore provides a pharmaceutical preparation for use by injection into the skin, wherein the pharmaceutical preparation is preparable by a production method comprising the steps of providing a blood sample collected from an organism, which blood sample is (1) a whole blood sample or (2) a whole blood sample from which erythrocytes have been depleted, providing a vessel or container, said vessel or container having an internal surface, and contacting said blood sample with said vessel or a container, wherein
(a) said production method further comprises the step of incubating said blood sample in said vessel or container for an incubation time, and optionally removing cellular constituents of said blood sample after said incubation,
(b) said blood sample comprises exosomes, and said production method further comprises the steps of concentrating said exosomes and optionally removing cellular constituents of said blood sample after said concentration, or the step of isolating said exosomes, or
(c) said production method further comprises the step of avoiding incubation of said blood sample, and the step of removing cellular constituents of said blood sample contacted with said vessel or container.

This embodiment may also be described as a method of treating a patient in need of such treatment by injection of a pharmaceutical preparation into the skin, wherein the pharmaceutical preparation is preparable by a production method comprising the following steps: providing a blood sample collected from an organism, which blood sample is (1) a whole blood sample or (2) a whole blood sample from which erythrocytes have been depleted, providing a vessel or container, said vessel or container having an internal surface, and contacting said blood sample with said vessel or a container, wherein
(a) said production method further comprises the step of incubating said blood sample in said vessel or container for an incubation time, and optionally removing cellular constituents of said blood sample after said incubation,
(b) said blood sample comprises exosomes, and said production method further comprises the steps of concentrating said exosomes and optionally removing cellular constituents of said blood sample after said concentration, or the step of isolating said exosomes, or
(c) said production method further comprises the step of avoiding incubation of said blood sample, and the step of removing cellular constituents of said blood sample contacted with said vessel or container.

Additionally, this embodiment may also be described as a method of preparing a pharmaceutical preparation for use by injection into the skin, wherein the method comprises the following steps: providing a blood sample collected from an organism, which blood sample is (1) a whole blood sample or (2) a whole blood sample from which erythrocytes have been depleted, providing a vessel or container, said vessel or container having an internal surface, and contacting said blood sample with said vessel or a container, wherein
(a) said production method further comprises the step of incubating said blood sample in said vessel or container for an incubation time, and optionally removing cellular constituents of said blood sample after said incubation,
(b) said blood sample comprises exosomes, and said production method further comprises the steps of concentrating said exosomes and optionally removing cellular constituents of said blood sample after said concentration, or the step of isolating said exosomes, or
(c) said production method further comprises the step of avoiding incubation of said blood sample, and the step of removing cellular constituents of said blood sample contacted with said vessel or container.

The present invention also provides a pharmaceutical preparation for use as described above (1) in the treatment of ageing or (2) as an anti-ageing agent.

It also provides a pharmaceutical preparation for use as described above in the treatment of
(a) a disorder caused by oxidative damage, DNA damage, impaired DNA repair, impaired cell division, excessive inflammation, a pathogenic polarisation of immune processes, or excessive cell death, or
(b) a disorder that is mimicked by a disorder of a genetically altered mouse that has at least one mutation in a gene encoding a protein of the Nucleotide Excision Repair pathway, said mutation causing a premature ageing phenotype as compared to a mouse lacking said mutation, or
(c) an age-related disorder or a disorder whose incidence increases with age in a greater than linear fashion, or
(d) a disorder having an effect on mechanical parameters of the skin or a disorder caused by collagen damage and/or elastin damage, senescence, telomere shortening, impaired expression of antioxidant enzymes or impaired activity of antioxidant enzymes
(which is preferably a use in the treatment of ageing or as an anti-ageing agent).

The present invention further provides a pharmaceutical preparation for use as described above in the treatment a disorder selected from the group consisting of Lichen sclerosus et atrophicus (LSA), Ehlers-Danklos Syndrome, Elastosis actinica, Elastoidosis cutanea nodularis et cystica and Elastosis perforans serpiginosa. In these disorders elasticity of the skin and/or the connective tissue is impaired.

"Disorders" as used herein refers to disturbances in normal function or appearance and includes diseases, complications and conditions.

The use by injection into the skin or the use in the above treatment is in the following sometimes referred to as "the use according to the present invention". Accordingly the pharmaceutical preparation for use by injection into the skin or for use in the above treatment is sometimes referred to as "the pharmaceutical preparation for use according to the present invention"

With regard to the above item (a), preferably said oxidative damage is damage by reactive oxygen species. Said disorder caused by DNA damage is preferably a disorder caused by UV-dependent DNA damage. Preferably said disorder caused by UV-dependent DNA damage is selected from a disorder caused by pyrimidine dimers, basal-cell carcinoma, squamous-cell carcinoma and melanoma. It is preferred that said disorder caused by impaired DNA repair is a disorder caused by deficient Nucleotide Excision Repair. Preferably said disorder caused by impaired cell division is a disorder associated with (more preferably caused and/or or mimicked by) impaired division of nucleus pulposus cells. In another preferred embodiment said disorder caused by excessive inflammation is a non-orthopaedic disorder, a disorder not involving the nervous system and/or a disorder not involving the eye. It is preferred that said disorder caused by excessive inflammation is different from one, more than one, or all of the following disorders: rheumatism, arthritis, rheumatoid arthritis, juvenile rheumatoid arthritis, psoriatic arthritis, Bechterew arthritis, osteoarthritis, back symptoms, neuroorthopaedic disorders, joint disorders, intervertebral disc disorders, spinal disorders, nerve root disorders, tendon disorders, loss of cartilage, neurodermitis and alopecia. Preferably said pathogenic polarisation of immune processes is a preponderance of Type 1 immune processes. Preferably said Type 1 immune processes are Tₕ1 and/or M1 processes, and more preferably they are Tₕ1 and M1 processes. "Preponderance of Type 1 immune processes" includes the meaning "preponderance of Type 1 over Type 2 immune processes", wherein said Type 2 immune processes are preferably Tₕ2 and/or M2 processes and more preferably Tₕ2 and M2 processes. "Preponderance of Type 1 immune processes" also includes the meanings "excessive Type 1 immune processes" and "impaired other immune processes", e.g. "impaired Type 2 immune processes" (with preferred embodiments as set out above). Said cell death is preferably apoptosis.

With regard to the above item (b), said mutation in said genetically altered mouse is preferably in the Ercc1 gene. More preferably it is Ercc1^{-/-} or Ercc1^{-/Δ}.

With regard to the above item (c), preferably said incidence increases exponentially with age.

With regard to the above item (d), preferably said disorder caused by collagen damage and/or elastin damage is selected from loose skin, dryness and wrinkling. A disorder caused by collagen damage and/or elastin damage may also be generally described as skin ageing.

It has now been found that a preparation prepared according to the adjusted production method of the present specification is capable of surprisingly improving skin parameters that can be assessed by a suction elasticity measurement using a Cutometer®, when being injected into the skin, in particular elasticity.

Parameters measured by a Cutometer® (such as the Cutometer® Dual MPA580) are as follows:
R-Parameters
   R0: Represents the passive behaviour of the skin to force (firmness), looks at the maximum amplitude.
   R1: Ability of the skin to return to its original state.
   R2: Gross elasticity, the higher the value the more elastic the curve.
   R3: Maximum amplitude of the last suction curve after repeated suction. "Tiring effects" (fatigue) of the skin are visible, as the amplitude increases with each new suction.
   R4: Last minimum amplitude compared to the first curve, "tiring effects" of the skin are visible, as the ability of redeformation decreases with each new suction.
   R5: Net elasticity, the higher the value, the more elastic is the skin.
   R6: Portion of the visco-elasticity on the elastic part of the curve. The smaller the value the higher the distensibility of the elastine fibres.
   R7: Portion of the elasticity compared to the complete curve, the higher the value, the more elastic the skin.
   R8: Skin recovery, the closer the value is to R0, the better is the ability of the skin to return to its original state.
   R9: Represents tiring effects of the skin after repeated suction and release of the skin. The smaller R9, the smaller the tiring effects.
F-Parameters
   F0, F1: These areas are deducted from the total area. A completely elastic material will show no area at all, the closer the value to 0, the more elastic the material.
   F2: Area above the upper envelope-curve showing tiring effects after repeated suction.
   F3: Area within the envelope curves, represents the skin fatigue.
   F4: Area under the upper envelope-curve. The smaller F4 the more the skin resists to the suction (skin firmness).
Q-Parameters
   Q0: Maximum recovery area, will decrease with increased firmness of the skin.
   Q1: Elastic recovery, will increase with more elasticity of the skin.
   Q2: Viscous recovery
   Q3: Viscoelastic recovery (overall elasticity), will increase with more elasticity of the skin.

Herein, R0 is considered to be the most important parameter (the lower the value, the higher is the elasticity). R5 is considered to be the second most important parameter.

Moreover it has been surprisingly found that a preparation prepared according to the production method of the present specification has, in addition to the improvement in skin parameters, a number of other effects that are relevant for various aspects of the (biological) ageing process. Since all of these effects are relevant for delaying, retarding, lessening, halting and/or reversing changes that accumulate in organisms over time, it can be concluded that such a preparation affects ageing as such and is therefore effective in the treatment of ageing or as an anti-ageing agent. More specifically, such a preparation counteracts the harmful effects of UV radiation on cells, has a stronger anti-inflammatory effect in older patients than in younger patients, contains an even more favourable ratio anti-inflammatory to inflammatory components in older patients than in younger patients, induces a shift in immune system function from inflammatory to regenerative and anti-inflammatory, stimulates an anti-apoptotic pathway and increases cell division, which serves as the basis of the present invention. The preparation prepared according to the production method of the present specification or the adjusted production method of the present specification is thus a pharmaceutical preparation, and it may be used as a novel means for treating, preventing, inhibiting or mitigating ageing or in interfering with its biological causes.

The pharmaceutical preparation for use according to the present invention is preparable by a production method which comprises: providing a liquid collected from an organism, which liquid comprises cellular constituents of blood, providing a vessel or containment means, said vessel or containment means having an internal surface, and contacting said liquid with said vessel or containment means, wherein (a) said production method further comprises the step of incubating said liquid in said vessel or containment means for an incubation time, and optionally removing cellular constituents of said liquid after said incubation, (b) said liquid comprises exosomes, and said production method further comprises the steps of concentrating said exosomes and optionally removing cellular constituents of said liquid after said concentration, or the step of isolating said exosomes, or (c) said production method further comprises the step of avoiding incubation of said liquid, and the step of removing cellular constituents of said liquid contacted with said vessel or containment means. Preferably said production method comprises: providing a blood sample collected from an organism, providing a vessel or container, said vessel or container having an internal surface, and contacting said blood sample with said vessel or a container, wherein (a) said production method further comprises the step of incubating said blood sample in said vessel or container for an incubation time, and optionally removing cellular constituents of said blood sample after said incubation, (b) said blood sample comprises exosomes, and said production method further comprises the steps of concentrating said exosomes and optionally removing cellular constituents of said blood sample after said concentration, or the step of isolating said exosomes, or (c) said production method further comprises the step of avoiding incubation of said blood sample, and the step of removing cellular constituents of said blood sample contacted with said vessel or container. After carrying out these method steps, a pharmaceutical preparation is present, and its efficacy results from carrying out these method steps. According to its alternative (a) the method results in a conditioned blood sample. Efficacy is in particular advantageous when the relationship between said incubation time and said internal surface is in accordance with the following equation: t = f ^{∗} A, wherein t designates the incubation time, A designates the internal surface and f is smaller than or equal to 0.5 h/cm². However efficacy is not dependent on incubation, as explained further below. Therefore according to alternative (b) incubation may be carried out or avoided, and according to alternative (c) incubation is avoided.

For example during the incubation step, efficacious components are induced in the liquid (preferably blood sample), in particular due to the activity of cells therein. Therefore preferably said liquid is a blood sample, which is a whole blood sample. However, the blood sample may also be a fraction of whole blood. For example erythrocytes, being cells that lack a nucleus and thus gene expression ability, may be absent from the blood sample. Therefore said blood sample is alternatively a whole blood sample from which erythrocytes have been depleted (preferably completely or substantially completely, but it is alternatively envisaged that only part of the erythrocytes have been depleted), for example a buffy coat or PRP (platelet-rich plasma). It is unnecessary to, and preferred not to, add any external stimulators or activators. Before the providing step, the production method of the present specification or the adjusted production method of the present specification preferably additionally includes the step of collecting said liquid (preferably blood sample, more preferably whole blood sample) from said organism.

Said organism may or may not suffer from any of the above-mentioned disorders and cells in that organism may or may not exhibit the above-mentioned phenotype.

The pharmaceutical preparation prepared according to the production method of the present specification has been found to counteract the harmful effects of UV radiation on cells. The cell count in cells irradiated with a combination of UV-A and UV-B radiation has been found to be higher in the presence of the pharmaceutical preparation prepared according to the production method of the present specification than in its absence. The magnitude of his effect was dependent on the incubating step. Therefore incubation of the liquid (preferably blood sample, more preferably whole blood sample) leads to the formation of components efficacious in promoting the survival and/or proliferation of UV-irradiated cells, which is relevant for one aspect of ageing.

In this respect the effect of the pharmaceutical preparation prepared according to the production method of the present specification, namely to rescue DNA damage caused by UV, is the same as that of a functional Nucleotide Excision Repair system. Therefore, without being bound to theory it may be concluded that the pharmaceutical preparation for use according to the present invention stimulates Nucleotide Excision Repair. More generally, it may be concluded that the pharmaceutical preparation for use according to the present invention has an effect on a disorder caused by impaired DNA repair or accumulation of DNA damage, which is relevant for another aspect of ageing.

Accordingly it may be assumed that the pharmaceutical preparation for use according to the present invention is useful in treating a disorder caused by insufficient Nucleotide Excision Repair. Several models exist that are deficient in Nucleotide Excision Repair, for example Ercc1^{-/-} mice. This model shows a premature ageing phenotype and is considered to reflect normal ageing, both in mice and in other organisms, such as humans. It shows age related pathologies in coat condition, kyphosis, gait, tremors, ataxia, dystonia and grip strength. Kyphosis has further implications on osteoporosis. Ataxia, dystonia and tremors are neurodegenerative disorders and have an impact on neurodegeneration and associated disorders like Alzheimer's disease, mild cognitive impairment, cerebrovascular disease, Parkinson's disease and amyotrophic lateral sclerosis. Gait and grip strength have an impact on muscle wasting. Moreover, neurodegeneration and associated disorders are usually linked to neuroinflammation. It can be concluded that the anti-inflammatory action of the pharmaceutical preparation for use according to the present invention is also useful in the treatment of neuroinflammation, neurodegeneration and associated disorders, an effect relevant for yet another aspect of ageing.

Moreover, the pharmaceutical preparation prepared according to the production method of the present specification stimulates the NF-κB pathway, which is known to have anti-apoptotic effects, in particular through inducing the expression of a number of genes whose products can inhibit apoptosis (see e.g. Karin & Lin (2002) Nat Immunol 3, 221). Therefore the conclusion may be drawn that it can be used in the treatment of a disorder caused by excessive cell death, which is relevant for an additional aspect of ageing.

The pharmaceutical preparation prepared according to the production method of the present specification has also been found to increase cell division. Accordingly it may be concluded that it has an effect on a disorder caused by impaired cell division. Since senescence and ageing may be caused by impaired cell division, this finding may contribute to explaining the general effect of the pharmaceutical preparation in ageing or age-related disorders or as an anti-ageing agent. For example, in the age-related degeneration of intervertebral discs as a whole, the degeneration of cells of the intervertebral disc is relevant. An increased division of cells of the intervertebral disc (nucleus pulposus) may rejuvenate the intervertebral discs and may help prevent disorders of the intervertebral discs such as slipped discs in which a lack of sufficient cell division and renewal may play a role.

The pharmaceutical preparation for use according to the present invention may afford, for example, a simple, cost effective and/or rapid production. By carrying out a series of easy steps in the production and without the need for special or complicated equipment and materials, in a minimum of steps and a few hours, a ready-to-use pharmaceutical preparation is realised without having to add any substances foreign to the body during production or such other substances which will have to separated again later in the production. By using exclusively a body's own substances, in this manner, an especially body-compatible agent is produced.

With regard to alternative (a) of the adjusted production method of the present specification, it is preferred that the relationship between said incubation time and said internal surface is in accordance with the following equation: t = f ^{∗} A, wherein t designates the incubation time, A designates the internal surface and f is smaller than or equal to 0.5 h/cm². Preferably f is smaller than or equal to 0.45 h/cm², smaller than or equal to 0.4 h/cm², smaller than or equal to 0.35 h/cm², smaller than or equal to 0.3 h/cm², smaller than or equal to 0.25 h/cm², smaller than or equal to 0.24 h/cm², smaller than or equal to 0.23 h/cm², and in particular smaller than or equal to 0.22 h/cm². This is useful for ensuring that the pharmaceutical preparation has no undesired inflammatory potential. Particularly preferred are values for f in the range of 0.04 to 0.25 h/cm², 0.05 to 0.24 h/cm², 0.06 to 0.23 h/cm² and in particular 0.07 to 0.22 h/cm², whereas in some instances ranges from 0.1 to 0.22 h/cm², 0.12 to 0.22 h/cm² or 0.14 to 0.22 h/cm² may also be used.

It is known that whole blood, and conditioned whole blood, contains exosomes. Exosomes are small vesicles secreted from cells into their environment. Exosomes are for example contained in biological liquids such as serum, urine, saliva, peritoneal-, cerebrospinal- and synovial liquids. Most types of cells looked at are able to secrete exosomes. The secretion occurs through release through/from the cell's plasma membrane. Depending on the cell type in which they are generated, exosomes contain inter alia a variable combination of proteins. In the following, the term "exosomes" preferably additionally comprises other extracellular vesicles (EV).

Conditioning, as described above, leads to additional formation of exosomes and other applications substances. However, it is not always necessary to condition a liquid (preferably blood sample). Since exosomes have been surprisingly found by the present inventors to exhibit a number of relevant effects (such as proliferation of cells in culture, drop in systemic CRP levels), exosomes may be concentrated or isolated after conditioning, but alternatively exosomes existing in the blood may be concentrated or isolated without conditioning in order to achieve beneficial effects.

Accordingly, with regard to alternative (b) of the adjusted production method of the present specification, it is preferred that the production method, before the step of concentrating or isolating said exosomes, further comprises the step of incubating said liquid (preferably blood sample) in said vessel or containment means for an incubation time, or the step of avoiding incubation of said liquid (blood sample). Concentrating or isolating is useful to further increase the efficacy.

Since also in the absence of incubation the pharmaceutical preparation prepared according to the adjusted production method of the present specification has been surprisingly found by the present inventors to exhibit a number of relevant effects (such as promoting cell survival after UV irradiation), incubation may be avoided in certain embodiments, in accordance with alternative (c) of the adjusted production method of the present specification. In this alternative, the step of removing cellular constituents of the liquid (preferably blood sample) is always carried out. Such a step of removing cellular constituents is often beneficial in all alternatives of the adjusted production method of the present specification.

With regard to all alternatives of the adjusted production method of the present specification, the step of removing cellular constituents is preferably a step of removing the erythrocytes (in particular in the case when said liquid comprising cellular constituents of blood is whole blood), the platelets (in particular in the case when said liquid comprising cellular constituents of blood is whole blood or whole blood from which erythrocytes have been depleted) or the entirety of cellular constituents (in particular in the case when said liquid comprising cellular constituents of blood is whole blood or whole blood from which erythrocytes have been depleted. It is most preferred that the step of removing cellular constituents is a step of removing the entirety of cellular constituents in the case when said liquid comprising cellular constituents of blood is whole blood). Such a separation may be achieved by centrifugation, such as a short centrifugation at a low relative centrifugal force (e.g. about 10 minutes at 1000 g) or by filtration.

In alternatives (a) and (b) of the adjusted production method of the present specification, the step of removing cellular constituents of said liquid is optional, and alternatively it is also envisaged to include a step of refraining from removing cellular constituents of said liquid (or refraining from removing those cellular constituents with specific desired functions). In particular it is envisaged to refrain from removing the erythrocytes, the platelets or the entirety of cellular constituents.

Preferably the adjusted production method of the present specification further comprises the step of reducing the volume of said liquid and/or the pharmaceutical preparation prepared according to the adjusted production method of the present specification is in dry form (in particular powder form).

Preferred ranges for the internal surface are 10 to 300 cm², 15 to 200 cm², 20 to 150 cm², 25 to 140 cm², 30 to 130 cm², 35 to 120 cm² and in particular 40 to 110 cm².

It is preferred that said blood sample is a whole blood sample, according to embodiment (1) of the adjusted production method of the present specification.

Preferably the injection is carried out at a depth of less than 3 mm, 2.5 mm or less, 2 mm or less, 1.5 mm or less or 1 mm or less. Preferred depth ranges are 0.1 mm to less than 3 mm, 0.5 mm to less than 3 mm, 1 mm to less than 3 mm, 1 to 2.5 mm, 1 to 2 mm or 1 to 1.5 mm.

It is preferred that the injection into the skin is an injection into the dermis (i.e. an intradermal injection) or subcutis (i.e. a subcutaneous injection).

Preferably the above use/treatment involves one or more subsequent injections, wherein a time interval between consecutive injections is 1 day to 52 weeks, 2 days to 42 weeks, 3 days to 30 weeks, 4 days to 24 weeks, 5 days to 18 weeks, 6 days to 12 weeks, 1 to 8 weeks, in particular 1 to 6 weeks, 1 to 4 weeks, 1 to 3 weeks, 1 to 2 weeks, 2 to 3 weeks or 2. In particular a total of at least 3 injections is preferred, most preferably at time intervals of 2 weeks between the first and the second injection and between the second and the third injection, or preferably between any two consecutive injections.

In order to achieve the maximum effect, it is preferred to avoid storage before using the pharmaceutical preparation preparable according to the production method of the present specification or the adjusted production method of the present specification.

Preferably the organism referred to above in the context of the pharmaceutical preparation for use according to the present invention is a human being. Preferably said human being is at least 30, 35, 40, 45, 48, 50, 55, 60 or 65 years old, or 30 to 65, 35 to 60 or 40 to 55 years old.

In a preferred pharmaceutical preparation for use according to the present invention (a) the level of IL-6 is 2000 pg/ml or less (preferably 1500 pg/ml or less, 1000 pg/ml or less, 800 pg/ml or less, 700 pg/ml or less, 600 pg/ml or less and in particular 500 pg/ml or less) (b) the ratio of the levels of IL-1Ra and IL-6, each measured in pg/ml, is 3 or more, 5 or more, 7 or more, 10 or more, 15 or more, 20 or more, 25 or more, 30 or more, 40 or more and in particular 50 or more, (c) the level of IL-1Ra is 200 pg/ml or more, 300 pg/ml or more, 400 pg/ml or more, 500 pg/ml or more, 600 pg/ml or more, 800 pg/ml or more, 1000 pg/ml or more, 1200 pg/ml or more, 1400 pg/ml or more, 1600 pg/ml or more, 1800 pg/ml or more and in particular 2000 pg/ml or more, (d) the ratio of the levels of IL-1Ra and IL-1, each measured in pg/ml, is 10 or more, 20 or more, 30 or more, 40 or more, 50 or more, 60 or more, 70 or more, 80 or more, 90 or more, 100 or more, 150 or more, 200 or more, 300 or more, 500 or more, 700 or more and in particular 1000 or more, or (e) the pharmaceutical preparation is free from added hyaluronic acid. IL-6 is an important factor for the skin, and also for the liver.

Preferably the pharmaceutical preparation for use according to the present invention comprises exosomes.

Preferably exosomes contained in the pharmaceutical preparation for use according to the present invention have been generated during the above-mentioned incubation, if any, of the liquid (preferably blood sample, more preferably whole blood sample) collected from the organism in a vessel or containment means.

The average diameter of the exosomes in an exosome-containing pharmaceutical preparation for use according to the present invention, as established by means of a transmission electron microscope, is preferably between 30 and 200 nm, in particular between 50 and 190 nm, between 70 and 180 nm, between 90 and 160 nm or between 100 and 150 nm. Exosomes of this size are the basis for an especially high efficacy, larger vesicle sizes may be indicative of conglomerates that contain damaged exosomes and aggregates. However, larger exosomes may also be functional, in particular with regard to shifting the balance from Type 1 immune responses to Type 2 immune responses. This applies to exosomes with a diameter range of 200 to 5000 nm or 100 to 800 nm. These larger exosomes may be obtained by differential centrifugation.

The production method of the present specification or the adjusted production method of the present specification preferably further comprises the step of concentrating or isolating the exosomes after the incubation, if any, in order to further increase its efficacy.

Such a concentrating or isolating step may lead to two or more pharmaceutical preparations prepared according to the production method of the present specification or the adjusted production method of the present specification. These two or more pharmaceutical preparations correspond to the fractions obtained after performing such a concentrating or isolating step.

Concentrating or isolating the exosomes may for example be realised through centrifugation at 2,000 to 1,000,000 g, 10,000 to 800,000 g, 20,000 to 600,000 g, 50,000 to 400,000 g, 80,000 to 200,000 g and in particular 100,000 g, as such accelerations are especially suitable to concentrate or isolate exosomes, depending on their sizes. Such a centrifugation is preferably conducted for at least 10 min, for at least 30 min, especially for at least 60 min. The pellet formed by the centrifugation then contains the exosomes. Preferably the concentrated or isolated exosomes are then taken up in a fluid (preferably a buffered solution such as PBS, or alternatively e.g. plasma or serum). Optionally they are then filtrated, for example through a 0.2 µm filter.

The present invention also conceives a pharmaceutical preparation for use according to the present invention that does not contain exosomes. However, a preferred pharmaceutical preparation for use according to the present invention does comprise exosomes, and in particular it may consist of exosomes.

In case serum or plasma is contained in the containing pharmaceutical preparation for use according to the present invention (which is preferred in certain cases), such serum or plasma preferably contains cytokines and/or growth factors.

Preferably the pharmaceutical preparation for use according to the present invention does not comprise a corticosteroid, since this may impair the efficacy of the pharmaceutical preparation, in particular due to inhibition of its senescence-rescuing effect and/or anti-apoptotic effect.

Incubation of the liquid (preferably blood sample, more preferably whole blood sample) is preferably carried out for an incubation time of 5 min to 22 hours, 10 min to 20 hours, 15 min to 18 hours, 30 min to 16 hours, 45 min to 15 hours, 1 to 14 hours, 2 to 13 hours, 3 to 12 hours, 4 to 11 hours, 5 to 10 hours or 6 to 9 hours.

The incubation is preferably carried out at a temperature of 0°C to 45°C, in particular at temperatures of 10°C to 43°C, 20°C to 41°C, 30°C to 40°C, 35°C to 39°C, 36°C to 38°C or 37°C. These temperatures ensure best efficacy.

Preferably the incubation is carried out in the absence of added heparin. More preferably it is carried out in the absence of any added anticoagulant.

Suitable vessels or containment means (preferably containers) for carrying out the production method of the present specification or the adjusted production method of the present specification are for example hypodermic needles, syringes, tubes such as vacuum tubes or test tubes, microtiter plates, syringes and transfusion bags. The vessel or containment means may e.g. have a diameter of 0.4 to 5 cm, 0.9 to 4 cm or 1.4 to 3.5 cm, and/or a length of 3 to 30 cm, 5 to 20 cm, 7 to 15 cm or 8 to 12 cm. Preferably, the vessel or containment means is cylindrical. A preferred vessel or containment means has a volume of 1 ml to 1000 ml, 3 ml to 750 ml, 5 ml to 500 ml, 7 ml to 300 ml, 8 ml to 200 ml, 9 ml to 150 ml, 10 ml to 100 ml, 11 ml to 80 ml and in particular 12 ml to 70 ml, however it may also have a volume of 15 ml to 65 ml, 20 ml to 60 ml, 25 ml to 50 ml or 30 to 40 ml. preferably the ratio of internal surface and volume is 0.01 to 10 cm²/ml. Preferred volumes of the liquid (preferably blood sample) are 0.5 ml to 900 ml, 1 ml to 700 ml, 2 ml to 400 ml, 3 ml to 300 ml, 6 ml to 200 ml, 7 ml to 150 ml, 8 ml to 100 ml, 9 ml to 80 ml and in particular 10 ml to 60 ml, however it may also have a volume of 11 ml to 55 ml, 12 ml to 50 ml, 15 ml to 40 ml or 20 to 30 ml.

The vessel or containment means preferably include(s) a surface for contacting the liquid (preferably blood sample, more preferably whole blood sample) that comprises glass, plastic, corundum or quartz or a combination thereof. A preferred plastic is selected from the group consisting of polystyrene, polycarbonate, polyethylene and polypropylene. Preferably the surface for contacting the liquid of a vessel or containment means creates a fully enclosed space. Preferred vessels or containment means have one or more of the following characteristics: symmetrical about a plane, symmetrical about an axis and cylindrical.

According to a preferred embodiment the vessel or containment means contain(s) macroscopic particles, microscopic particles or nanoparticles and during the incubation the liquid (preferably blood sample) is in contact with said particles. For the purposes of the present application, macroscopic particles are defined as particles that are visible when viewed with the naked eye, microparticles are defined as particles that are too small to be visible when viewed with the naked eye but are visible when viewed with a microscope, and nanoparticles are defined as particles that are too small to be visible when viewed with a microscope (and that are preferably larger than 1 nm). Such particles serve the purpose of enlarging the surface for contacting the liquid (blood sample) (e.g. by another 0.3 to 90 cm², 2 to 80 cm², 5 to 70 cm², 10 to 60 cm², 20 to 50 cm² or 30 to 40 cm²) and can have the shape of spheres, granulates, powder, gels or wool. Preferred materials are glass, plastic, corundum, quartz, gold and clay mineral (e.g. kaolin). Especially preferred are glass spheres. The surface of the particles can optionally be modified, for example by incubation with a caustic agent such as 50% v/v chromosulphuric acid with subsequent repeated rinsing. As stated above, the surface of such particles is not considered when calculating the "internal surface" of the vessel or containment means.

Preferably the pharmaceutical preparation for use according to the present invention is for use by injection to the same organism from whom said liquid (preferably blood sample, more preferably whole blood sample) has been collected. In this case it is preferably an organism that suffers from one or more of the above-mentioned disorders. Thus the pharmaceutical preparation for use according to the present invention is preferably an autologous pharmaceutical preparation and not an allogeneic one, especially for reasons of safety. When describing the present invention as the above-mentioned method of treating a patient, this means that the organism and the patient are identical.

In a preferred embodiment the pharmaceutical preparation for use according to the invention is for use is in a combination therapy with one or more other effective agents. Preferred other effective agents are agents effective in the treatment of ageing or as an anti-ageing agent (especially in the treatment of one or more of the disorders mentioned above), hyaluronic acid or botulinum toxin, or a combination thereof. Particularly preferred is a combination of the pharmaceutical preparation for use according to the invention, hyaluronic acid and botulinum toxin.

### Examples

### Example 1: Proliferation of cells of nucleus pulposus origin

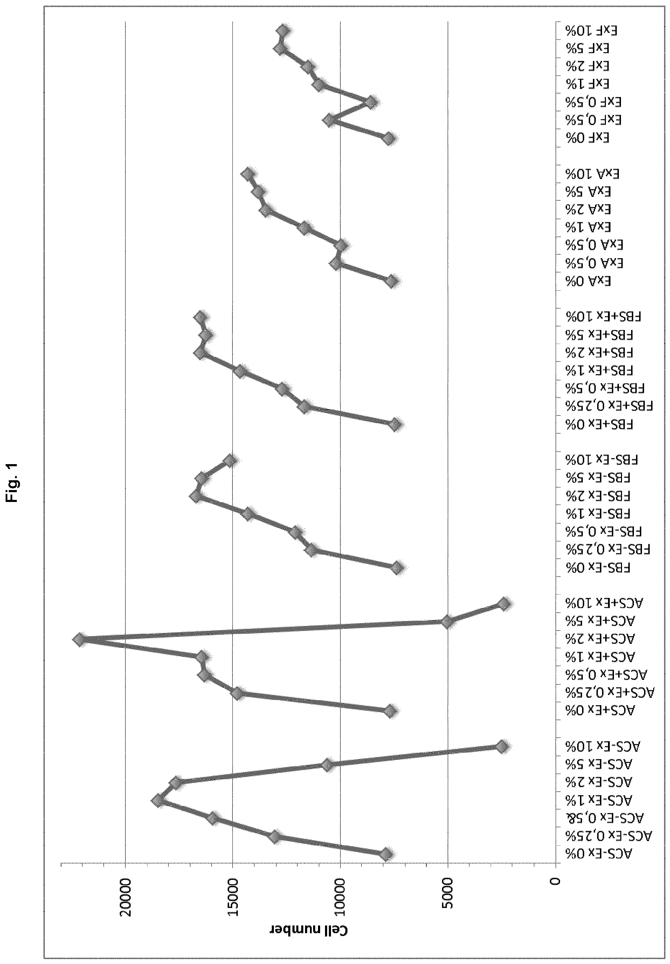

The proliferation of nucleus pulposus cells was tested in the presence of various supplements: Human conditioned serum ("ACS"), which is a pharmaceutical preparation prepared according to the production method of the present specification, with and without exosomes; foetal bovine serum ("FBS") with and without exosomes; and exosomes isolated from ACS and from FBS (see Figure 1).

Cells of nucleus pulposus origin were plated in a 96 well plate (8000 cells per well) with DMEM/F12 medium and PenStrep 1%. Cell culture medium was supplemented with full ACS with exosomes ("ACS+Ex"), ACS without exosomes ("ACS-Ex"), exosomes from ACS ("ExA"), full FBS with exosomes ("FBS+Ex"), FBS without exosomes ("FBS-Ex") and exosomes from FBS ("ExF"). Cell division was assessed photometrically after 24 h with an XTT assay in an ELISA reader. Exosomes were separated from ACS and FBS by 2 h centrifugation of 30 ml ACS and 30 ml FBS, respectively, at 100,000 g and resuspension of pellet in 3 ml of PBS each.

It was found that both ACS and FBS promoted cell division. Cells divided best with lower ACS concentrations (1% to 2%). At higher concentrations of ACS (5%, 10%), both in the presence and in the absence of exosomes, an apparent decrease in cell number was observed, but this may reflect detachment of cells or cell conglomerates when cell culture medium was washed off and then replaced by XTT staining medium. This may result in false readings because only attached cells can contribute to the signal observed in this XTT assay. The highest proliferation was seen in the presence of ACS with exosomes. FBS showed no comparable apparent decrease in cell number at higher concentrations such as 5% and 10%, but was less efficient overall in promoting cell division. In the experiments performed with FBS, exosomes did not have a major effect on cell proliferation. Isolated exosomes of FBS origin and of ACS origin promoted cell division dose-dependently.

Consequently the pharmaceutical preparation preparable according to the production method of the present specification increases cell division.

### Example 2: Stimulation of an anti-apoptotic pathway

The stimulation the NF-κB pathway, which has an anti-apoptotic effect, was tested by using a GFP/Luciferase reporter system by using the following samples: a pharmaceutical preparation prepared according to the production method of the present specification, which did not comprise isolating or concentrating exosomes generated during the incubation, and wherein the pharmaceutical preparation was a serum and the incubation time was 6 h ("ACS") and a control using control serum for which the production method of the present specification was not carried out ("Control"). The NF-κB pathway was stimulated with various concentrations of IL-1β. In this experiment cells of chondrocytic origin were genetically altered with a DNA construct consisting of a basic CMV promoter driving a luciferase gene and a preceding 4 fold binding motif (TRE) for NF-κB proteins. Any induction of the NF-κB pathway in these cells is therefore visible as an enhanced luciferase activity (relative light units (RLU)) which can be quantified.

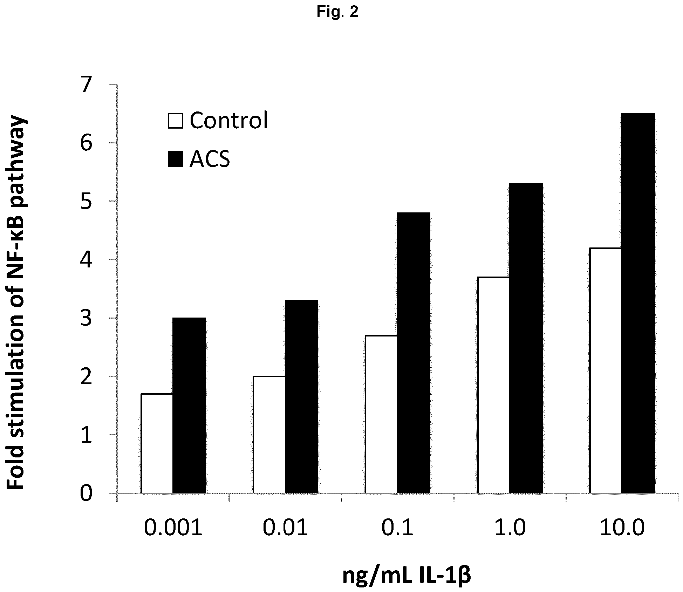

The results are shown in the following table and in Figure 2.

In Figure 2, the left bar in each group represents the Control and the right bar represents ACS.

| **IL-1β (ng/ml)** | **0.001** | **0.01** | **0.1** | **1** | **10** |
|---|---|---|---|---|---|
| Fold-increase in Control | 1.7 | 2.0 | 2.7 | 3.7 | 4.2 |
| Fold-increase in ACS | 3.0 | 3.3 | 4.8 | 5.3 | 6.5 |

It is apparent that the NF-κB pathway was stimulated by IL-1β in a dose-dependent manner. The pharmaceutical preparation prepared according to the production method of the present specification ("ACS") enhanced this stimulation, over the whole range of tested IL-1β concentrations.

Thus it can be concluded that a pharmaceutical preparation preparable according to the production method of the present specification stimulates an anti-apoptotic pathway.

### Example 3: UV radiation of cells

In the first series of experiments, nucleus pulposus cells were plated in 24 well plates in the absence of serum and in the presence of a pharmaceutical preparation prepared according to the production method of the present specification, which was in the form of a serum ("EOT"). EOT concentrations were 0.05%, 0.1%, 0.2%, 0.5% and 1%. One part of the EOT samples was not incubated, but processed immediately ("EOT0"). The other part of the EOT samples was incubated for six hours ("EOT6").

Six hours after plating the cells, cells were irradiated with UV light (Herolab transilluminator FT-28/312, 6 tubes cat. nr. 29 84 100, 15 W, 312 nm). The emission maximum was 312 nm. The duration of irradiation was 80 s. Cells were grown for another 2 days at 37°C, 5% CO₂.

Cells were detached with 500 µl of 1% trypsin and counted in a Casy counter. The cell numbers per ml were as follows:

| **Additive** | **UV** | **no UV** |
|---|---|---|
| no Serum | 1.20E+03 | 5.92E+05 |
| | | |
| EOT0 0.05% | 2.00E+03 | 6.34E+05 |
| EOT0 0.1% | 1.50E+03 | 5.23E+05 |
| EOT0 0.2% | 2.00E+03 | 9.74E+05 |
| EOT0 0.5% | 2.50E+03 | 1.48E+06 |
| EOT0 1% | 3.50E+03 | 1.00E+06 |
| | | |
| EOT6 0.05% | 1.50E+03 | 7.44E+05 |
| EOT6 0.1% | 1.20E+04 | 1.24E+06 |
| EOT6 0.2% | 8.00E+03 | 1.70E+06 |
| EOT6 0.5% | 1.00E+04 | 1.70E+06 |
| EOT6 1% | 1.35E+04 | 6.30E+05 |

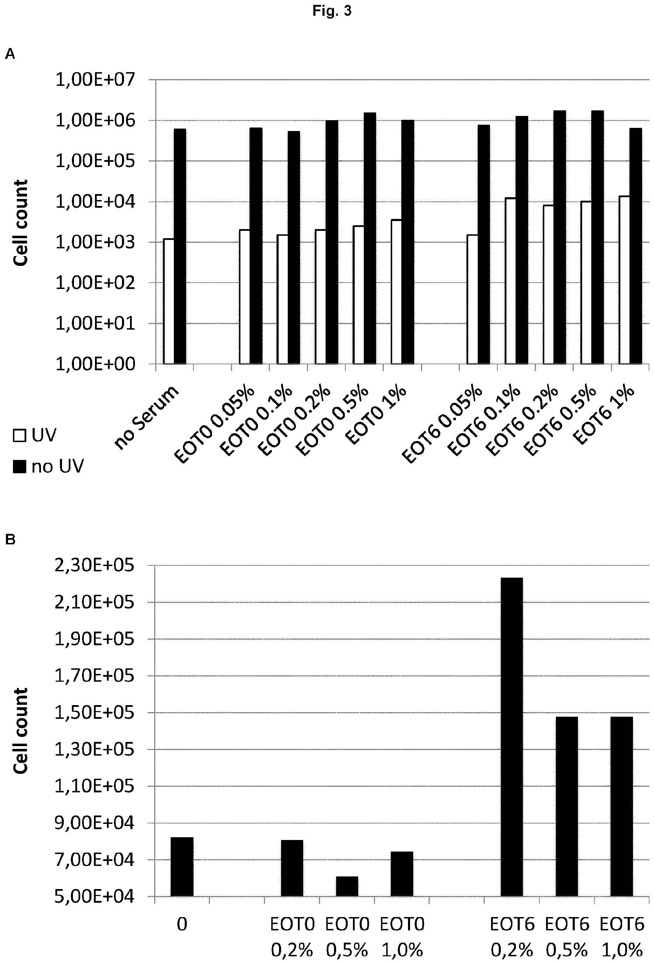

A graph of the data is shown in Figure 3A.

It was found that UV irradiation strongly decreased the cell count in the absence of serum. The presence of EOTO did not have a large effect. In contrast, the presence of EOT6 increased the cell counts by a factor of about 10, except for the lowest concentration of 0.05%.

It may be concluded that the pharmaceutical preparation prepared according to the production method of the present specification counteracts the harmful effects of UV radiation. This effect was dependent on incubation, i.e. on the formation of efficacious components during the period of incubation.

A second series of experiments confirmed these results. In this series, the non-irradiated controls were omitted. The results (cells/ml) are shown in the following table and in Figure 3B.

| **Additive** | **UV** |
|---|---|
| no Serum | 8.22E+04 |
| | |
| EOT0 0.2% | 8.07E+04 |
| EOT0 0.5% | 6.09E+04 |
| EOT0 1.0% | 7.44E+04 |
| | |
| EOT6 0.2% | 2.23E+05 |
| EOT6 0.5% | 1.48E+05 |
| EOT6 1.0% | 1.48E+05 |

Again it was seen that the pharmaceutical preparation prepared according to the production method of the present specification counteracts the harmful effects of UV radiation, and that this effect was dependent on the incubation step.

### Example 4: Drop in systemic CRP levels

Human subjects of different age groups (20 to 45 years, 48 to 58 years, 60 to 83 years) received intramuscular or intra-articular injections of the pharmaceutical preparation prepared according to the production method of the present specification, which comprised isolated exosomes. Exosomes were separated from the conditioned blood sample by 2 h centrifugation at 100,000 g and the pellet was resuspended in PBS. The volume of PBS was one tenth of the serum volume. The subjects were intramuscularly administered 1 ml in each case. The total number of subjects was 22. Subjects each received a single injection, and CRP levels were measured before and about two weeks after the injection.

In each case the level of C-reactive protein (CRP) was determined by high sensitivity CRP ELISA ("hsCRP").and compared to the level before the treatment. In order to be able to compare the results, the relative drop in CRP level was calculated in each case. It was found that the median relative drop was least pronounced in the 20 to 45 year age group, more pronounced in the 48 to 58 year age group and most pronounced in the 60 to 83 year age group.

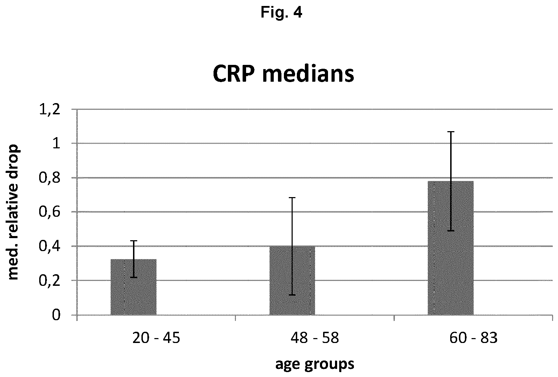

The results are shown in Figure 4.

It can be concluded that the pharmaceutical preparation prepared according to the production method of the present specification decreases inflammation, as shown by the CRP levels, and thus counteracts a possible cause of ageing. It does even more so in older patients than in younger patients.

### Example 5: Age-dependence of the ratio of anti-inflammatory to inflammatory components

It was examined whether the age of a human being has an influence on the composition of the pharmaceutical preparation prepared according to the production method of the present specification.

To this end, in a series of in vitro experiments blood samples were collected from human subjects and subjected to the production method of the present specification. The concentrations of IL-1β and IL-1Ra were determined after the incubating step.

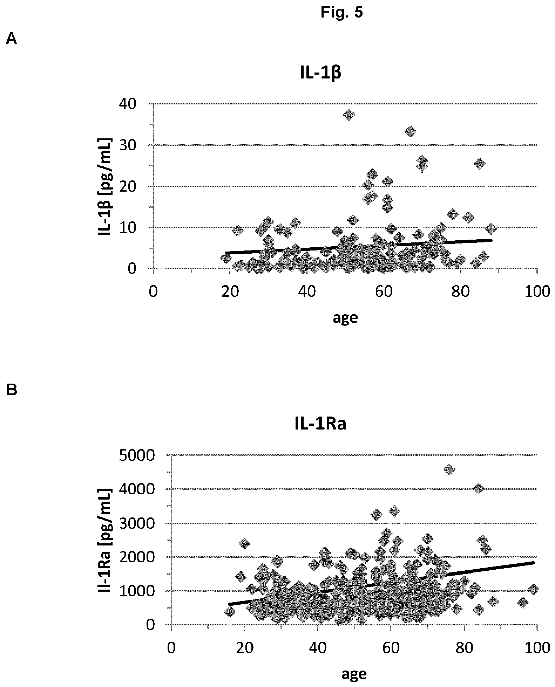

It was found that the concentration of IL-1β (inflammatory component) after incubation was not statistically significant dependent on age. The results are shown in Figure 5A (total number of subjects: 165).

In contrast, the concentration of IL-1Ra (anti-inflammatory component) after incubation was higher in older patients than in younger patients. The results are shown in Figure 5B (total number of subjects: 368).

The conclusion is that the pharmaceutical preparation prepared according to the production method of the present specification as an anti-inflammatory effect and thus counteracts a possible cause of ageing. This effect is even stronger in older patients than in younger patients, as shown by the fact that the concentration of IL-1Ra after incubation increases with age, but the concentration of IL-1β does not.

### Example 6: Shift in immune system function

Four human volunteers each received four intramuscular injections of 4 ml of the pharmaceutical preparation prepared according to the production method of the present specification, which was in the form of an Autologous Conditioned Serum (1x per week).

Whole blood was drawn from the subjects at week 0, week 2 and week 4 and subjected to in vitro testing: The production of IL-2 and IFN-γ in the whole blood were tested in vitro under 24 h stimulation with 5.7 µg/ml PHA. The CRP levels in blood were additionally determined by high sensitivity C-reactive protein ELISA ("hsCRP").

It was found that the injections led to a strong decrease of the capability of the whole blood to produce IL-2 and IFN-γ, as shown in the following table. hsCRP was decreased.

| **Mean values** | **IL-2 (pg/ml)** | **IFN-γ (pg/ml)** | **hsCRP (pg/ml)** |
|---|---|---|---|
| week 0 | 88.9 | 375.5 | 2.2 |
| week 2 | 70.5 | 80.2 | 1.9 |
| week 4 | 35.7 | 82.8 | 1.7 |
| | | | |

| **SD values** | **IL-2 (pg/ml)** | **IFN-γ (pg/ml)** | **hsCRP (pg/ml)** |
|---|---|---|---|
| week 0 | 91.9 | 233.6 | 2.4 |
| week 2 | 68.4 | 49.8 | 0.4 |
| week 4 | 39.5 | 69.9 | 1.2 |

The decrease in Type 1 cytokines (IL-2 and IFN-γ) means that the injections had induced a shift of the immune system from inflammatory to regenerative/anti-inflammatory. Therefore an imbalance in immune processes due to a preponderance of Type 1 immune processes may be treated.

From this it can be concluded that the pharmaceutical preparation prepared according to the production method of the present specification or the adjusted production method of the present specification counteracts a possible cause of ageing.

### Example 7: Elasticity measurements after injection into facial skin

By injection into the facial skin 21 patients with a loss of facial skin and elasticity (age 35 to 55 years) were treated. Patients were injected with a pharmaceutical preparation prepared according to the adjusted production method of the present specification, wherein the cellular constituents of the blood sample had been removed after the incubation, in the form of a serum ("ACS"). The incubation time t was 6 h and the internal surface A was 41 cm². Being defined as t/A, f was thus 0.146.

The total study duration was 24 weeks. The visits were as follows: Screening visit (week -2, that is 2 weeks before enrolment), enrolment visit (week 0), week 2, week 4, week 8, week 12 and week 24. The following table shows an overview of the visits. Activities performed are marked with an "x":

| | Week | | | | | | |
|---|---|---|---|---|---|---|---|
| | -2 | 0 | 2 | 4 | 8 | 12 | 24 |
| | screening | enrolment | | | | | |
| Written informed consent | x | | | | | | |
| Demographic data | x | | | | | | |
| Verification of inclusion/exclusion criteria | x | | | | | | |
| Medical history | x | | | | | | |
| Physical examination | x | | | | | | |
| ACS intradermal microinjections | | x | x | x | | (x) | |
| Assessments | x | x | x | x | x | x | x |
| Lab test and vital signs | x | | | | | | |
| Recording of adverse events | x | x | x | x | x | x | x |

Exclusion criteria were severe chronic skin diseases e.g. psoriasis, atopic eczema (neurodermatitis) or other autoimmune skin diseases, skin cancer in the anamnesis, systemic diseases with skin involvement (SLE), pre-treatment with laser, botulinum toxin or hyaluronic acid, pregnancy, severe diet in the last 3 months or nutritional supplementary during the study duration of 24 weeks, acute infection, acne.

Patients underwent a series of three injections of ACS at week 0, week 2 and week 4. A fourth injection was given at week 12 for the majority of patients.

About 2 ml of ACS were injected intradermally (at a depth of 1 to less than 3 mm) in total at each session. Injections were done manually on both malar regions (≤ 1 ml per region) in a grid of 1 cm distance between neighbouring injection sites. At each cheek an area of about 4 cm by 5 cm was treated by 20 injections.

For the primary efficacy evaluation the suction principle was used in the skin. With a Cutometer® MPA 580 (Courage & Khazaka, Cologne, Germany) 12 parameters describing the viscoelastic properties of the skin were assessed over 24 weeks 7 times (week -2, week 0 and weeks 2, 4, 8, 12 and 24). In the case of non-efficacy measured at week 12, an additional ACS administration was performed.

The primary efficacy variable was gained in skin elasticity measured with the Cutometer®. Additional variables were corneometry (measurement of hydration), Global Aesthetic Improvement Scale (GAIS) scale (extent of improvement/no change/worse) self-assessment of attractiveness and a questionnaire.

The results were as follows:

| | **Baseline** | | **Week 12** | | **Difference** | | |
|---|---|---|---|---|---|---|---|
| | **mean** | **SD** | **mean** | **SD** | **mean** | **SD** | **p value** |
| Patient age | 46,71 | 5,87 | | | | | |
| Corneometry - right cheek | 44,11 | 12,61 | 53,46 | 10,90 | 9,35 | 16,69 | 0,02 |
| Corneometry - left cheek | 41,09 | 12,51 | 55,93 | 11,58 | 14,84 | 12,74 | <0,01 |
| Corneometry - both cheeks | 42,61 | 12,13 | 54,70 | 10,43 | 12,09 | 13,83 | <0,01 |
| Cutometer R0 - both cheeks | 0,34 | 0,03 | 0,24 | 0,05 | -0,10 | 0,05 | <0,01 |
| Cutometer R1 - both cheeks | 0,10 | 0,03 | 0,06 | 0,02 | -0,04 | 0,02 | <0,01 |
| Cutometer R2 - both cheeks | 0,69 | 0,07 | 0,74 | 0,06 | 0,04 | 0,06 | 0,01 |
| Cutometer R3 - both cheeks | 0,39 | 0,04 | 0,29 | 0,06 | -0,10 | 0,05 | <0,01 |
| Cutometer R4 - both cheeks | 0,18 | 0,04 | 0,11 | 0,04 | -0,06 | 0,03 | <0,01 |
| Cutometer R5 - both cheeks | 0,36 | 0,06 | 0,45 | 0,10 | 0,09 | 0,08 | <0,01 |
| Cutometer R6 - both cheeks | 0,39 | 0,05 | 0,48 | 0,07 | 0,09 | 0,09 | <0,01 |
| Cutometer R7 - both cheeks | 0,26 | 0,04 | 0,30 | 0,05 | 0,04 | 0,04 | <0,01 |
| Cutometer R8 - both cheeks | 0,23 | 0,03 | 0,18 | 0,04 | -0,06 | 0,04 | <0,01 |
| Cutometer R9 - both cheeks | 0,05 | 0,01 | 0,05 | 0,01 | 0,00 | 0,01 | 0,05 |
| Cutometer F0 - both cheeks | 0,05 | 0,01 | 0,05 | 0,01 | -0,01 | 0,01 | <0,01 |
| Cutometer F1 - both cheeks | 0,05 | 0,01 | 0,04 | 0,01 | -0,01 | 0,01 | <0,01 |
| Cutometer Q0 - both cheeks | 67,65 | 6,97 | 48,10 | 10,01 | -19,55 | 9,89 | <0,01 |
| Cutometer Q1 - both cheeks | 0,61 | 0,07 | 0,65 | 0,06 | 0,04 | 0,06 | 0,01 |
| Cutometer Q2 - both cheeks | 0,45 | 0,06 | 0,51 | 0,07 | 0,06 | 0,05 | <0,01 |
| Cutometer Q3 - both cheeks | 0,16 | 0,02 | 0,15 | 0,03 | -0,02 | 0,03 | 0,02 |
| Cutometer ratio R1/R4 - both cheeks | 0,58 | 0,05 | 0,55 | 0,05 | -0,02 | 0,06 | 0,12 |
| Cutometer ratio R3/R9 - both cheeks | 7,50 | 1,07 | 5,93 | 0,92 | -1,57 | 1,12 | <0,01 |

| | | | | | | | |
|---|---|---|---|---|---|---|---|
| SD: standard deviation Difference: visit 5 minus baseline p value: paired t-Test It was noted that even small differences can lead to a statistically significant result, in case the standard deviation is small. | | | | | | | |

Comparing week 12 and baseline, the R0 value was greatly decreased by about 30%, from 0.34 to 0.24 (significant). This can be considered to be a difference representative of an age difference of two decades. The R5 value was greatly increased by about 25% (significant).

Without being bound by theory, the significant increase in elasticity might be due to an activation of fibroblasts.

Thus it can be concluded that effects last at least 12 weeks.

| | | **Baseline** | **Week 2** | **Week 4** | **Week 8** | **Week 12** |
|---|---|---|---|---|---|---|
| Total sum | **Number** | **21** | **21** | **21** | **21** | **21** |
| | **Percent** | **100,00%** | **100,00%** | **100,00%** | **100,00%** | **100,00%** |
| **Skin phenotype** | | | | | | |
| Type II | Number | 11 | | | | |
| | % | 52,38 | | | | |
| Type III | Number | 7 | | | | |
| | % | 33,33 | | | | |
| Type IV | Number | 3 | | | | |
| | % | 14,29 | | | | |
| | | | | | | |

| **Skin condition** | | | | | | |
|---|---|---|---|---|---|---|
| Normal | Number | 8 | | | | |
| | % | 38,10 | | | | |
| Dry | Number | 10 | | | | |
| | % | 47,62 | | | | |
| Oily | Number | 3 | | | | |
| | % | 14,29 | | | | |
| | | | | | | |

| **Skin sensitivity** | | | | | | |
|---|---|---|---|---|---|---|
| Not sensitive | Number | 14 | | | | |
| | % | 66,67 | | | | |
| Sensitive | Number | 7 | | | | |
| | % | 33,33 | | | | |
| | | | | | | |

| **GAIS right cheek (investigator)** | | | | | | |
|---|---|---|---|---|---|---|
| Worse | Number | | 0 | 0 | 0 | 0 |
| | % | | 0,00 | 0,00 | 0,00 | 0,00 |
| No change | Number | | 19 | 14 | 13 | 10 |
| | % | | 90,48 | 66,67 | 61,90 | 47,62 |
| Improved | Number | | 2 | 7 | 8 | 11 |
| | % | | 9,52 | 33,33 | 38,10 | 52,38 |
| Much improved | Number | | 0 | 0 | 0 | 0 |
| | % | | 0,00 | 0,00 | 0,00 | 0,00 |
| | | | | | | |

| **GAIS left cheek (investigator)** | | | | | | |
|---|---|---|---|---|---|---|
| Worse | Number | | 0 | 0 | 0 | 0 |
| | % | | 0,00 | 0,00 | 0,00 | 0,00 |
| No change | Number | | 18 | 12 | 8 | 7 |
| | % | | 85,71 | 57,14 | 38,10 | 33,33 |
| Improved | Number | | 3 | 9 | 13 | 14 |
| | % | | 14,29 | 42,86 | 61,90 | 66,67 |
| Much improved | Number | | 0 | 0 | 0 | 0 |
| | % | | 0,00 | 0,00 | 0,00 | 0,00 |
| | | | | | | |

| **GAIS right cheek (test person)** | | | | | | |
|---|---|---|---|---|---|---|
| Worse | Number | | 0 | 0 | 0 | 1 |
| | % | | 0,00 | 0,00 | 0,00 | 4,76 |
| No change | Number | | 14 | 10 | 5 | 8 |
| | % | | 66,67 | 47,62 | 23,81 | 38,10 |
| Improved | Number | | 6 | 10 | 15 | 12 |
| | % | | 28,57 | 47,62 | 71,43 | 57,14 |
| Much improved | Number | | 1 | 1 | 1 | 0 |
| | % | | 4,76 | 4,76 | 4,76 | 0,00 |
| | | | | | | |

| **GAIS left cheek (test person)** | | | | | | |
|---|---|---|---|---|---|---|
| Worse | Number | | 0 | 0 | 0 | 1 |
| | % | | 0,00 | 0,00 | 0,00 | 4,76 |
| No change | Number | | 15 | 11 | 5 | 8 |
| | % | | 71,43 | 52,38 | 23,81 | 38,10 |
| Improved | Number | | 6 | 10 | 16 | 12 |
| | % | | 28,57 | 47,62 | 76,19 | 57,14 |
| Much improved | Number | | 0 | 0 | 0 | 0 |
| | % | | 0,00 | 0,00 | 0,00 | 0,00 |
| | | | | | | |

| **Self-assessment of attractiveness** | | | | | | |
|---|---|---|---|---|---|---|
| Rather unattractive | Number | 1 | 0 | 0 | 0 | 0 |
| | % | 4,76 | 0,00 | 0,00 | 0,00 | 0,00 |
| Moderately attractive | Number | 15 | 14 | 16 | 17 | 12 |
| | % | 71,43 | 66,67 | 76,19 | 80,95 | 57,14 |
| Rather attractive | Number | 5 | 6 | 5 | 4 | 9 |
| | % | 23,81 | 28,57 | 23,81 | 19,05 | 42,86 |
| Quite attractive | Number | 0 | 1 | 0 | 0 | 0 |
| | % | 0,00 | 4,76 | 0,00 | 0,00 | 0,00 |

### Example 8: Proliferation of cells of nucleus pulposus origin

A pharmaceutical preparation was prepared according to the adjusted production method of the present specification in the form of a serum ("ACS"). The incubation time t was 6 h and the internal surface A was 41 cm². Being defined as t/A, f was thus 0.146.

It is established that ACS is capable of promoting cell proliferation in vitro. Here different components of ACS were assessed for promoting proliferation of nucleus pulposus (NP) cells in vitro.

Nucleus pulposus cells were plated out in a 96 well cell culture plate at a density of ca. 4500 cells/well. Cells were then fed with various components of ACS: ACS was fractionated via a size exclusion column (IZON qEV, cf. qEV Size Exclusion Columns for EV separation and purification http://www.izon.com/assets/SideColumnPDFs/qEV-Brochure-April-15.pdf). This column is capable of separating exosomes (EV) from proteins and other components. The largest particles (EV) are eluted first, followed by gradually smaller ones. The exact fraction number may depend on factors such as buffer, temperature and the identity of the applied fluid (e.g. serum, plasma or other fluids). However there will be at least 2 separate peaks that have pro-proliferative activity.

Briefly, qEV columns were equilibrated with cell culture medium (DMEM, Gibco); 500 µl of ACS was applied to columns and fractionated as follows. The first 1.5 ml were discarded, the following liquid was collected in 350 µl fractions. Fractions were used to feed cells and numbers of cells were determined by a CCK-8 ("Cell Counting Kit-8") assay.

CCK-8 provides a sensitive colorimetric assay for the determination of cell viability. A highly water-soluble tetrazolium salt, WST-8, is reduced by dehydrogenase activities in cells to give an orange-colour formazan dye, which is soluble in the tissue culture media. The absorption at 450 nm is proportional to active mitochondria in cells, see http://www.dojindo.eu.com/store/p/456-Cell-Counting-Kit-8.aspx. The amount of the formazan dye, generated by the activities of dehydrogenases in cells, is directly proportional to the number of living cells.

The results document that exosomes are capable of promoting NP cell proliferation. The baseline was the cell proliferation with DMEM only.

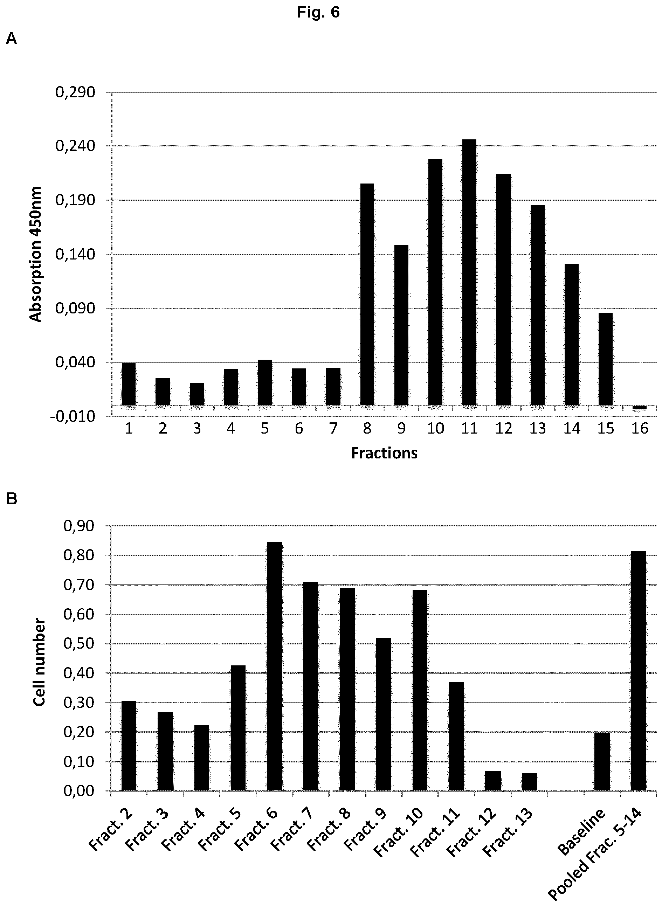

In the first CCK-8 assay (Fig. 6 A) exosomes eluted in fractions 8 and 9. Starting with fraction 10 the proteins (including the growth factors from ACS) begin to elute and form another peak leading to cell proliferation. Fractions from 16 contained components which did not stimulate cell growth.

In the second CCK-8 assay (Fig. 6 B) exosomes eluted in fractions 5 to 8, and proteins eluted in fractions 9 to 11. Fractions from 12 contained components which did not stimulate cell growth. Pooled fractions 5 to 14 combine exosomes (EVs) and proteins.

The conclusion is that EV from ACS promote cell proliferation. Proteins from ACS also promote cell proliferation. ACS contains two groups of components which independently promote cell proliferation: EV (Exosomes) and proteins, such as growth factors. A third group of components in ACS reduces cell proliferation and elutes with small components. Note: The exact fraction number of the components depends on factors such as: column size, buffer and temperature. The sequence of components does not change under non-denaturing conditions. Pooling of fractions containing EV and fractions containing proteins such as growth factors may work synergistically.

Therefore it can be concluded that ACS contains 2 groups of components that independently promote cell proliferation in vitro.

### Example 9: Cell survival after UV irradiation in vitro

A pharmaceutical preparation was prepared according to the adjusted production method of the present specification in the form of a serum ("ACS"). The incubation time t was 6 h and the internal surface A was 41 cm². Being defined as t/A, f was thus 0.146.

ACS is capable of promoting cell proliferation in vitro. This experiment assessed ACS and other blood preparations for promoting survival of nucleus pulposus cells in vitro after UV irradiation.

Briefly, nucleus pulposus cells were plated out in a 96 well cell culture plate at a density of ca. 3000 cells/well and kept at 37°C, 5% CO₂. Cells were fed with 10%, 5%, 2.5%, 1.25% and 0.63% each of foetal bovine serum (FBS), ACS, platelet-rich plasma (PRP), plasma (identical to ACS but without incubation) and platelet lysate (PL).

Half of such wells were irradiated for 30 min with high intensity UV radiation. Cells in each well were counted via IMAGER.

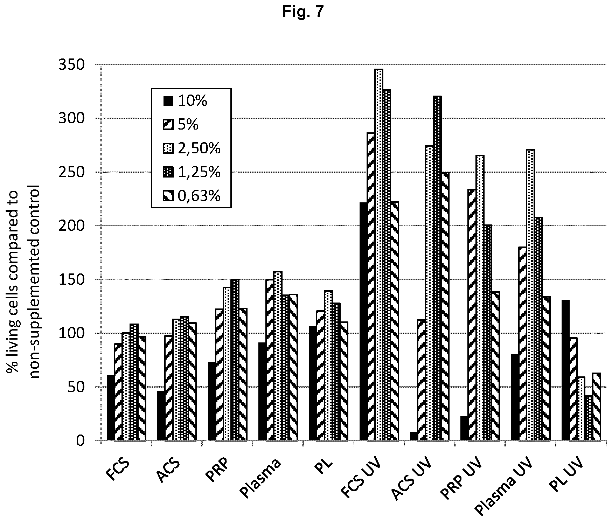

Figure 7 shows percentages of living cells compared to non-supplemented controls 24 hours after supplement addition and UV irradiation. The data are based on the mean of three 96 well plates.

In the absence of UV irradiation (left five columns) the various concentrations of supplements led to cell numbers in the range of about 50% to 150% of those of non-supplemented controls.

After UV irradiation the majority of non-supplemented cells died. Cell death occurred to a higher degree than in the absence of UV irradiation.

The supplements protected the cells to various degrees from UV irradiation induced cell death, depending on their concentration. This is shown by the percentages of living cells compared to the non-supplemented controls (right five columns), which were clearly above 100%. The maximum protection was achieved with 2.5% and 1.25% of FCS and 1.25% of ACS. The effects of PRP and plasma were less pronounced. A comparison between plasma and ACS showed that in certain concentrations incubation has a clear effect, but that a certain effect can also be achieved in the absence of incubation. Platelet lysate was not effective in this experiment.

Conclusion: UV irradiation causes net cell death in vitro. Supplementation with blood derived preparations clearly increases survival as compared to non-supplemented cells.

### Preferred Embodiments

### Items

1. A pharmaceutical preparation for use by injection into the skin, wherein the pharmaceutical preparation is preparable by a production method comprising the steps of providing a liquid collected from an organism, which liquid comprises cellular constituents of blood, providing a vessel or containment means, said vessel or containment means having an internal surface, and contacting said liquid with said vessel or containment means, wherein
   (a) said production method further comprises the step of incubating said liquid in said vessel or containment means for an incubation time, and optionally removing cellular constituents of said liquid after said incubation,
   (b) said liquid comprises exosomes, and said production method further comprises the steps of concentrating said exosomes and optionally removing cellular constituents of said liquid after said concentration, or the step of isolating said exosomes, or
   (c) said production method further comprises the step of avoiding incubation of said liquid, and the step of removing cellular constituents of said liquid contacted with said vessel or containment means.
2. The pharmaceutical preparation for use according to alternative (a) of item 1, wherein the relationship between said incubation time and said internal surface is in accordance with the following equation: t = f ^{∗} A, wherein t designates the incubation time, A designates the internal surface and f is smaller than or equal to 0.5 h/cm².
3. The pharmaceutical preparation for use according to alternative (b) of item 1, wherein said production method, before the step of concentrating or isolating said exosomes, further comprises the step of incubating said liquid in said vessel or containment means for an incubation time, or the step of avoiding incubation of said liquid.
4. The pharmaceutical preparation for use according to item 1, 2 or 3, wherein said liquid is a blood sample.
5. The pharmaceutical preparation for use according to item 4, which is (1) a whole blood sample or (2) a whole blood sample from which cells have been depleted.
6. The pharmaceutical preparation for use according to item 5, wherein said cells that have been depleted are erythrocytes.
7. The pharmaceutical preparation for use according to any of the above items, wherein the step of removing cellular constituents is a step of removing the erythrocytes, the platelets or the entirety of cellular constituents.
8. The pharmaceutical preparation for use according to any of the above items, wherein
   (a) said production method further comprises the step of reducing the volume of said liquid and/or
   (b) the pharmaceutical preparation is in dry form.
9. The pharmaceutical preparation for use according to any of the above items, wherein said injection is carried out at a depth of less than 3 mm.
10. The pharmaceutical preparation for use according any of the above items, wherein said injection into the skin is an injection into the dermis or subcutis.
11. The pharmaceutical preparation for use according to any of the above items, wherein the use involves one or more subsequent injections, and a time interval between consecutive injections is 1 day to 52 weeks.
12. The pharmaceutical preparation for use according to any of the above items (1) in the treatment of ageing or (2) as an anti-ageing agent.
13. The pharmaceutical preparation for use according to item 12 in the treatment of
   (a) a disorder caused by oxidative damage, DNA damage, impaired DNA repair, impaired cell division, excessive inflammation, a pathogenic polarisation of immune processes, or excessive cell death, or
   (b) a disorder that is mimicked by a disorder of a genetically altered mouse that has at least one mutation in a gene encoding a protein of the Nucleotide Excision Repair pathway, said mutation causing a premature ageing phenotype as compared to a mouse lacking said mutation, or
   (c) an age-related disorder or a disorder whose incidence increases with age in a greater than linear fashion, or
   (d) a disorder having an effect on mechanical parameters of the skin or a disorder caused by collagen damage and/or elastin damage, senescence, telomere shortening, impaired expression of antioxidant enzymes or impaired activity of antioxidant enzymes.
14. The pharmaceutical preparation for use according to item 13, wherein
   (a) said oxidative damage is damage by reactive oxygen species, said disorder caused by DNA damage is a disorder caused by UV-dependent DNA damage, said disorder caused by impaired DNA repair is a disorder caused by deficient Nucleotide Excision Repair, said disorder caused by impaired cell division is a disorder associated with impaired division of nucleus pulposus cells, said disorder caused by excessive inflammation is a non-orthopaedic disorder, a disorder not involving the nervous system and/or a disorder not involving the eye, said pathogenic polarisation of immune processes is a preponderance of Type 1 immune processes, or said cell death is apoptosis, or
   (b) said mutation in said genetically altered mouse is in the Ercc1 gene, or
   (c) said incidence increases exponentially with age, or
   (d) said disorder caused by collagen damage and/or elastin damage is selected from loose skin, dryness and wrinkling.
15. The pharmaceutical preparation for use according to any of the above items, wherein said organism is a human being.
16. The pharmaceutical preparation for use according to item 15, wherein said human being is at least 30 years old.
17. The pharmaceutical preparation for use according to any of the above items, wherein
   (a) the level of IL-6 is 2000 pg/ml or less,
   (b) the ratio of the levels of IL-1Ra and IL-6, each measured in pg/ml, is 3 or more,
   (c) the level of IL-1Ra is 200 pg/ml or more,
   (d) the ratio of the levels of IL-1Ra and IL-1, each measured in pg/ml, is 10 or more,
   (e) the pharmaceutical preparation is free from added hyaluronic acid.
18. The pharmaceutical preparation for use according to any of the above items, wherein said pharmaceutical preparation comprises exosomes.
19. The pharmaceutical preparation for use according to item 18, wherein exosomes have been generated during said incubation.
20. The pharmaceutical preparation for use according to any of items 18 to 19, wherein said production method further comprises the step of concentrating or isolating said exosomes after said incubation, and optionally taking up the concentrated or isolated exosomes in a fluid.
21. The pharmaceutical preparation for use according to any of the above items, which comprises serum or plasma.
22. The pharmaceutical preparation for use according to any of the above items, wherein said incubation is carried out
   (a) for an incubation time of 5 min to 22 hours,
   (b) at a temperature from 0°C to 45°C, and/or
   (c) in the absence of an added anticoagulant.
23. The pharmaceutical preparation for use according to any of the above items, wherein said vessel or containment means
   (a) has/have a volume of 1 ml to 1000 ml,
   (b) include(s) a surface for contacting the liquid, preferably blood sample, that comprises glass, plastic, corundum or quartz or a combination thereof and/or
   (c) contain(s) particles selected from the group consisting of macroscopic particles, microscopic particles and nanoparticles, and wherein during said incubation the liquid (preferably blood sample) is in contact with said particles.
24. The pharmaceutical preparation for use according to any of the above items, wherein said use is
   (a) by injection to the same organism from whom said liquid (preferably blood sample) has been collected and/or
   (b) in a combination therapy with one or more other effective agents.

## Claims

1. A pharmaceutical preparation for use in a method of therapeutically improving one or more skin parameters selected from elasticity of the skin, firmness of the skin, or hydration of the skin, wherein the use is by injection into the skin, wherein the pharmaceutical preparation is preparable by a production method comprising the steps of providing a liquid comprising cellular constituents of blood collected from a human organism, wherein said liquid is a blood sample, and providing a vessel or containment means, said vessel or containment means having an internal surface, and contacting said liquid with said vessel or containment means, wherein said production method further comprises the step of incubating said liquid in said vessel or containment means for an incubation time.

2. The pharmaceutical preparation for use according to claim 1, wherein said pharmaceutical preparation comprises exosomes.

3. The pharmaceutical preparation for use according to any one of claims 1 or 2, wherein said production method further comprises the step of removing the cellular constituents after said incubation.

4. The pharmaceutical preparation for use according to any of the above claims for therapeutically improving elasticity of the skin by activating fibroblasts.

5. The pharmaceutical preparation for use according to any one of claims 2 to 4, wherein said production method further comprises the step of concentrating said exosomes, and preferably further comprises removing the cellular constituents after said concentration.

6. The pharmaceutical preparation for use according to any of the above claims, wherein the blood sample is (1) a whole blood sample or (2) a whole blood sample from which cells have been depleted, wherein said cells that have been depleted are preferably erythrocytes.

7. The pharmaceutical preparation for use according to any one of claims 3 to 6, wherein the step of removing the cellular constituents is a step of removing the erythrocytes, the platelets or the entirety of the cellular constituents.

8. The pharmaceutical preparation for use according to any of the above claims, wherein said injection into the skin is an injection into the dermis or subcutis.

9. The pharmaceutical preparation for use according to any of the above claims, wherein the use involves one or more subsequent injections, and wherein the time interval between the consecutive injections is 1 day to 52 weeks.

10. The pharmaceutical preparation for use according to any of the above claims, wherein
(a) the level of IL-6 is 2000 pg/ml or less,
(b) the ratio of the levels of IL-1Ra and IL-6, each measured in pg/ml, is 3 or more,
(c) the level of IL-1Ra is 200 pg/ml or more,
(d) the ratio of the levels of IL-1Ra and IL-1, each measured in pg/ml, is 10 or more,
(e) the pharmaceutical preparation is free from added hyaluronic acid.

11. The pharmaceutical preparation for use according to any of the above claims, wherein the pharmaceutical preparation comprises serum or plasma.

12. The pharmaceutical preparation for use according to any of the above claims, wherein said incubation is carried out
(a) for an incubation time of 5 min to 22 hours,
(b) at a temperature from 0°C to 45°C, and/or
(c) in the absence of an added anticoagulant.

13. The pharmaceutical preparation for use according to any of the above claims, wherein said use is
(a) by injection to the same organism from whom said liquid (preferably blood sample) has been collected and/or
(b) in a combination therapy with one or more other effective agents.

14. A pharmaceutical preparation for use in a method of therapy of a collagen damage of the skin in patients in need thereof, wherein the use is by injection into the skin, wherein the pharmaceutical preparation is preparable by a production method comprising the steps of providing a liquid comprising cellular constituents of blood collected from a human organism, wherein said liquid is a blood sample, and providing a vessel or containment means, said vessel or containment means having an internal surface, and contacting said liquid with said vessel or containment means, wherein said production method further comprises the step of incubating said liquid in said vessel or containment means for an incubation time.

15. A pharmaceutical preparation for use in a method of therapy of an elastin damage of the skin in patients in need thereof, wherein the use is by injection into the skin, wherein the pharmaceutical preparation is preparable by a production method comprising the steps of providing a liquid comprising cellular constituents of blood collected from a human organism, wherein said liquid is a blood sample, and providing a vessel or containment means, said vessel or containment means having an internal surface, and contacting said liquid with said vessel or containment means, wherein said production method further comprises the step of incubating said liquid in said vessel or containment means for an incubation time.
